# EUROPÄISCHE PATENTSCHRIFT

(11) **EP 0 859 001 B1**
(45) Veröffentlichungstag und Bekanntmachung des Hinweises auf die Patenterteilung: **05.12.2001**
(21) Anmeldenummer: 98101934.2
(22) Anmeldetag: 05.02.1998
(51) Int. Cl.: C07D 263/34

(54) **Herstellung von Oxazolderivaten**
Preparation of oxazole derivatives
Préparation de dérivés d'oxazole

(30) Priorität: 13.02.1997 EP 97102339
(43) Veröffentlichungstag der Anmeldung: 19.08.1998
(73) Patentinhaber: F. HOFFMANN-LA ROCHE AG, 4070 Basel (CH)
(72) Erfinder: Behringer, Klaus, 4052 Basel (CH); Bonrath, Werner, 79115 Freiburg (DE); Pauling, Horst, 4103 Bottmingen (CH)
(74) Vertreter: Kellenberger, Marcus, Dr.

(56) Entgegenhaltungen:
- EP-A- 0 010 697
- EP-A- 0 492 233
- EP-A- 0 612 736
- EP-A- 0 770 604
- US-A- 3 222 374
- US-A- 4 026 901
- T. RINDERSPACHER ET AL.: HELVETICA CHIMICA ACTA, Bd. 43, Nr. 6, 1960, Seiten 1522-30, XP002065042

## Beschreibung

Die vorliegende Erfindung betrifft ein neues Verfahren zur Herstellung von 5-Cyano-4-niederalkyl-oxazolen. Diese Oxazole bilden eine wichtige Stoffgruppe. So ist beispielsweise 5-Cyano-4-methyl-oxazol ein wertvolles Zwischenprodukt bei der Synthese von Pyridoxin (Vitamin B₆).

Es wurden bereits einige Verfahren zur Herstellung von 5-Cyano-4-methyl-oxazol durch Dehydratisierung von 5-Carbamoyl-4-methyl-oxazol beschrieben. So gelingt diese Dehydratisierung beispielsweise in Gegenwart von Phosphorpentoxid [siehe Helv. Chim. Acta 43, 1522-1530 (1960)]. Die Nachteile dieses Verfahrens sind jedoch die geringe Produktausbeute, was vermutlich auf die bei dieser Reaktion sehr leicht auftretende Verkohlung zurückzuführen ist, und die Bildung von als Sondermüll geltendem Nebenprodukt Phosphat.

Eine Verbesserung dieses Verfahrens gelingt durch die Umsetzung von 5-Carbamoyl-4-methyl-oxazol mit Phosphorpentoxid in Gegenwart von Chinolin als Lösungsmittel [US-Patentschrift (USP) 3 222 374]. Auch dieses Verfahren weist jedoch Nachteile auf, die sich aus der Giftigkeit des Chinolins, seinem unangenehmen Geruch sowie seiner thermischen Instabilität ergeben. Zudem ist Chinolin ein relativ teures Lösungsmittel. Die Regenerierung des Chinolins, die erforderliche Verwendung von stöchiometrischen Mengen Phosphorpentoxid, die aufwendige Aufarbeitung der Folgeprodukte des Phosphorpentoxids (Phosphate) sowie deren umweltgerechte Entsorgung stellen noch weitere Probleme dar.

Ein weiteres bekanntes Verfahren zur Herstellung von 5-Cyano-4-methyl-oxazol besteht darin, das 5-Carbamoyl-4-methyl-oxazol mit einem Niederalkancarbonsäureanhydrid umzusetzen und das Reaktionsgemisch oder das aus diesem isolierte 4-Methyl-5-(N-niederalkanoyl-carbamoyl)-oxazol einer Pyrolyse zu unterwerfen [Europäische Patentpublikation (EP) 10 697]. Die pyrolytische Endstufe hat jedoch gewisse Nachteile, und zwar insbesondere auftretende Korrosionsprobleme mit den Reaktorwerkstoffen und die Bildung schwer rezyklisierbarer Nebenprodukte. Ferner stellt die hohe Temperatur, bei der die Pyrolyse erfolgen muss, einen Nachteil dar.

Das in USP 4 026 901 beschriebene Verfahren besteht darin, 5-Carbamoyl-4-methyl-oxazol katalytisch unter Erhitzen bei hoher Temperatur in Anwesenheit von Phosphorpentoxid auf einem festen Träger zu 5-Cyano-4-methyloxazol zu dehydratisieren. Nachteilig bei diesem Verfahren sind die Handhabung von 5-Carbamoyl-4-methyl-oxazol, insbesondere die im Vordergrund stehende Sublimation und damit die Feststoffdosierung des schwerflüchtigen Ausgangsmaterials, sowie wiederum die Bildung und umweltgerechte Entsorgung des Nebenproduktes Phosphat.

In der USP 4 772 718 wird ferner die einstufige Ueberführung von 4-Methyl-oxazol-5-carbonsäureethylester in 5-Cyano-4-methyl-oxazol beschrieben. Bei diesem Verfahren wird der entsprechende Oxazolester in Gegenwart von Ammoniak und einem Zirkonoxid- oder Hafniumoxidkatalysator in der Gasphase zu 5-Cyano-4-methyl-oxazol umgesetzt. Nachteilig ist hier jedoch die Verwendung eines relativ teuren Katalysators sowie - zwecks Erreichen einer optimalen Reaktionsführung - das Einhalten sehr enger Reaktionsbedingungen, u.a. einer nachteilig hohen Reaktionstemperatur. Der technische Aufwand ist dementsprechend hoch.

Die in der EP 492 233 beschriebene Gasphasendehydratisierung von 5-Carbamoyl-4-methyl-oxazol zu 5-Cyano-4-methyl-oxazol besitzt den Nachteil, dass die Umsetzung bei Reaktionstemperaturen von etwa 400°C bis etwa 500°C und bei einem hohen Druck, nämlich von etwa 50 bis etwa 300 kPa, durchgeführt werden muss. Wiederum handelt es sich um einen hohen technischen Aufwand.

Der vorliegenden Erfindung liegt nun die Aufgabe zugrunde, ein Verfahren zur Herstellung eines 5-Cyano-4-C₁₋₆-alkyl-oxazols durch Dehydratisierung eines 5-Carbamoyl-4-C₁₋₆-alkyl-oxazols zur Verfügung zu stellen, das die Nachteile der vorbekannten einschlägigen Verfahren nicht aufweist, und durch welches das 5-Cyano-4-C₁₋₆-alkyl-oxazol in kurzer Reaktionszeit, unter milden Reaktionsbedingungen und in hoher Ausbeute erhalten wird. Das erfindungsgemässe Verfahren ist dadurch gekennzeichnet, dass man die Dehydratisierung mit Siliziumtetrachlorid in Gegenwart eines Amins und in einem organischen Lösungsmittel durchführt. Das erfindungsgemäss verwendete Dehydratisierungsmittel Siliziumtetrachlorid ist relativ kostengünstig, und das im Verlauf der Dehydratisierung anfallende Siliziumdioxid nicht umweltproblematisch. Weiterhin vorteilhaft sind die guten Recyclisierungsraten der verwendeten Lösungsmittel und Basen (Amine).

Der Ausdruck "C₁₋₆-alkyl" bedeutet im Rahmen der vorliegenden Erfindung einen geradkettigen oder verzweigten Alkylrest mit 1 bis 6 Kohlenstoffatomen, z.B. Methyl, Ethyl, Propyl, Isopropyl, Butyl, Isobutyl, tert.Butyl, Pentyl oder Hexyl. Geradkettige Alkylreste, und insbesondere Methyl und Ethyl, vor allem Methyl, sind bevorzugt.

Als Amine sind sowohl aliphatische Amine als auch Stickstoff enthaltende heteroaromatische Verbindungen geeignet. Als aliphatische Amine eignen sich vor allem die tertiären aliphatischen Amine, insbesondere Trialkylamine mit geradkettigen oder verzweigten Alkylresten enthaltend 1 bis 10 Kohlenstoffatome. Beispiele solcher Alkylreste sind Methyl, Ethyl, n-Propyl, Isopropyl, n-Butyl, Isobutyl, tert.Butyl, Pentyl, Hexyl, Octyl und Decyl. Bei solchen Trialkylaminen können die Alkylreste gleich oder verschieden sein. Beispiele dieser Trialkylamin - und zugleich bevorzugte Trialkylamine - sind Triethylamin, Diisopropylethylamin und Tri(n-butyl)amin.

Bei den Stickstoff enthaltenden heteroaromatischen Verbindungen handelt es sich insbesondere um Heterozyklen, deren Ring mindestens ein Stickstoffatom aufweist. Beispiele sind Pyridin und Pyridinderivate, wie Picolin und Chinolin.

Als organische Lösungsmittel für das erfindungsgemässe Verfahren eignen sich insbesondere aliphatische und cyclische Ether, z.B. Diethylether und tert.Butylmethylether bzw. Tetrahydrofuran und Dioxan; aliphatische Nitrile, z.B. Acetonitril; aromatische Kohlenwasserstoffe, z.B. Toluen; sowie N-Methylpyrrolidon. Vorzugsweise wird tert.Butylmethylether oder Acetonitril als Lösungsmittel verwendet.

Zur Dehydratisierung wird das 5-Carbamoyl-4-niederalkyl-oxazol mit dem Dehydratisierungsmittel Siliziumtetrachlorid zweckmässigerweise bei Raumtemperatur oder höheren Temperaturen bis etwa 65°C, vorzugsweise bei Temperaturen von etwa 25°C bis etwa 60°C, besonders bevorzugt von etwa 45°C bis etwa 55°C, umgesetzt.

Das erfindungsgemässe Verfahren wird zweckmässigerweise durchgeführt, indem man das Siliziumtetrachlorid zu einer gut gerührten Lösung oder Suspension des 5-Carbamoyl-4-niederalkyl-oxazols und des Amins im Lösungsmittel zutropft. Die Umsetzung mit Siliziumtetrachlorid erfolgt zweckmässigerweise bei einem Molverhältnis Siliziumtetrachlorid : 5-Carbamoyl-4-niederalkyl-oxazol von etwa 0,6 : 1 bis etwa 3 : 1, vorzugsweise von etwa 0,7 : 1 bis etwa 0,75 : 1. Das Molverhältnis Amin : 5-Carbamoyl-4-niederalkyl-oxazol beträgt zweckmässigerweise von etwa 2,1 : 1 bis etwa 3,5 : 1, vorzugsweise von etwa 2,7 : 1 bis etwa 2,9 : 1. Je nach Lösungsmittel beträgt dessen Menge gegenüber der Menge eingesetzten 5-Carbamoyl-4-niederalkyloxazols (Oxazol) zweckmässigerweise von etwa 500 bis etwa 900 ml pro Mol Oxazol, vorzugsweise von etwa 550 bis etwa 650 ml pro Mol Oxazol. Zudem empfiehlt es sich, die Dehydratisierung unter einem Inertgas, z.B. Argon oder Stickstoff, durchzuführen, um möglichst Sauerstoff auszuschliessen. Auf diese Weise wird die Dehydratisierung normalerweise innert etwa 4 bis 10 Stunden, vorzugsweise innert etwa 5 bis 7 Stunden, beendet.

Die Aufarbeitung zur Isolierung des 5-Cyano-4-niederalkyl-oxazols kann durch gängige Verfahren der organischen Chemie, z.B. durch Destillation, erfolgen.

Wie oben erwähnt, besteht ein Vorteil des erfindungsgemässen Verfahrens darin, dass man das im Verlauf der Dehydratisierung anfallende Siliziumdioxid, das Lösungsmittel sowie das Amin nach erfolgter Reaktion rückgewinnen und recyclisieren kann. Ferner ist die Ausbeute an 5-Cyano-4-niederalkyl-oxazol hoch.

Die nachfolgenden Beispiele veranschaulichen das erfindungsgemässe Verfahren.

### Beispiel 1

### Allgemeine Arbeitsvorschrift für die Dehydratisierung von 5-Carbamoyl-4-methyl-oxazol zu 5-Cyano-4-methyl-oxazol

In einem mit Rührer, gekühlter Rührhülse, Thermometer, 50 ml-Tropftrichter (ohne Druckausgleich) und Dimroth-Kühler ausgestatteten 350 ml-Vierhalssulfierkolben wurden unter Argon 2,52 g (20 mmol) 5-Carbamoyl-4-methyl-oxazol (OXA) vorgelegt. Anschliessend wurden 60-80 ml eines Lösungsmittels und 60 mmol eines Amins als Base zugegeben. Danach wurden 22-60 mmol Siliziumtetrachlorid (SiCl₄) als Dehydratisierungsmittel unter gutem Rühren zur vorliegenden Suspension so zugetropft, dass die Innentemperatur im Kolben 45°C nicht überschreitete. Die Zutropfzeit betrug etwa 15 Minuten. Der Ansatz wurde anschliessend auf 60°C geheizt, wobei er sich von rötlich nach schwarzbraun verfärbte.

Die Ausbeute an 5-Cyano-4-methyl-oxazol (OXN) wurde jeweils durch Gaschromatographie (GC) ermittelt, und die Gehaltsbestimmungen erfolgten jeweils mittels eines internen Standards.

### a) Verwendung verschiedener Lösungsmittel

Diese Versuche wurden bei 60°C mit 6,07 g (60 mmol) Triethylamin als Base und 4,4 g (26 mmol) Siliziumtetrachlorid als Dehydratisierungsmittel (Molverhältnis SiCl₄:OXA = 1,3:1) durchgeführt. Die Reaktionszeit betrug verschiedentlich 2, 4 oder 5 Stunden. Die Ergebnisse der Versuche, in denen verschiedene Lösungsmittel in einer Menge von 60 oder 80 ml eingesetzt wurden, sind in der nachfolgenden Tabelle 1 zusammengefasst. Die Ausbeuten an OXN wurden durch GC ermittelt.

**Tabelle 1**

| Lösungsmittel | Menge Lösungsmittel | Reaktionszeit (Stunden) | Ausbeute an OXN |
|---|---|---|---|
| Tetrahydrofuran | 80 ml | 5 | 85% |
| Toluen | 60 ml | 5 | 31% |
| tert.Butylmethylether | 80 ml | 5 | 46% |
| Acetonitril | 60 ml | 4 | 95% |
| N-Methylpyrrolidon | 80 ml | 2 | 96% |

Diese Ergebnisse zeigen, dass für Dehydratisierung von OXA zu OXN Acetonitril bzw. N-Methylpyrrolidon besonders bevorzugt ist.

### b) Verwendung verschiedener Basen

Diese Versuche wurden bei 60°C mit 60 ml Acetonitril als Lösungsmittel und 4,4 g (26 mmol) Siliziumtetrachlorid als Dehydratisierungsmittel (Molverhältnis SiCl₄:OXA = 1,3:1) durchgeführt. Die Reaktionszeit lag im Bereich 3,5-5,5 Stunden. Die Ergebnisse der Versuche, in denen verschiedene Basen in einer Menge von 60 mmol, z.B. 6,07 g Triethylamin bzw. 11,1 g Tri(n-butyl)-amin, eingesetzt wurden, sind in der nachfolgenden Tabelle 2 zusammengefasst. Die Ausbeuten an OXN wurden durch GC ermittelt.

**Tabelle 2**

| Base | Reaktionszeit (Stunden) | Ausbeute an OXN |
|---|---|---|
| Triethylamin | 4 | 95% |
| Tri(n-butyl)amin | 3,5 | 94% |
| Pyridin | 5 | 90% |
| Chinolin | 5,5 | 94% |

Die Ergebnisse zeigen, dass gute Ausbeuten an OXN bei der Verwendung der obigen aliphatischen und aromatischen tertiären Amine erzielt wurden.

### c) Verwendung verschiedener Molverhältnisse

Diese Versuche wurden bei 60°C mit 80 ml Tetrahydrofuran als Lösungsmittel und 6,07 g (60 mmol) Triethylamin als Base durchgeführt, wobei die Reaktionszeit 5 oder 16 Stunden betrug. Die Ergebnisse der Versuche, in denen verschiedene Molverhältnisse SiCl₄:OXA und stets 2,52 g (20 mmol) OXA verwendet wurden, sind in der nachfolgenden Tabelle 3 zusammengefasst. Die Ausbeuten an OXN wurde durch GC ermittelt.

**Tabelle 3**

| Molverhältnis SiCl₄ : OXA | Reaktionszeit (Stunden) | Ausbeute an OXN |
|---|---|---|
| 3:1 | 5 | 92,5% |
| 1,5:1 | 16 | 94,5% |
| 1,3:1 | 5 | 85,0% |
| 1,1:1 | 5 | 84,5% |

Die Ergebnisse zeigen, dass für die Dehydratisierung von OXA ein Ueberschuss an SiCl₄ notwendig ist, was auf den Einfluss des Lösungsmittels (Tetrahydrofuran in diesem Fall) zurückzuführen ist.

### Beispiel 2

Diese Versuche wurden analog der in Beispiel 1 beschriebenen Arbeitsweise durchgeführt, allerdings ausgehend von 63,1 g (0,5 mol) OXA und unter Verwendung verschiedener Mengen der Base Tri(n-butyl)amin, des Lösungsmittels Acetonitril und des Dehydratisierungsmittels Siliziumtetrachlorid. Ferner wurde die Reaktionszeit variiert. Bei allen Versuchen wurde bei 55°C umgesetzt. Die Ergebnisse der Versuche sind in der nachfolgenden Tabelle 4 zusammengefasst. Die Ausbeuten an OXN wurden durch GC ermittelt. Die Mengenangaben für die Base und das SiCl₄ sind in Aequivalenten, bezogen auf die Menge des eingesetzten OXAs, angegeben.

**Tabelle 4**

| Menge Base (Aeq.) | Menge SiCl₄ (Aeq.) | Menge Lösungsmittel (ml) | Reaktionszeit (Stunden) | Ausbeute an OXN |
|---|---|---|---|---|
| 2,1 | 0,7 | 85 | 6 | 88 |
| 2,8 | 0,7 | 85 | 6 | 87 |
| 2,8 | 0,7 | 170 | 5 | 89 |
| 2,8 | 0,735∗ | 170 | 5,5 | 89 |
| 2,8 | 0,735∗ | 285 | 8 | 95 |

| | | | | |
|---|---|---|---|---|
| ∗ Die Menge an SiCl₄ wurde in Portionen von 0,7 und 0,035 Aequivalenten zugegeben. | | | | |

### Beispiel 3

In einem mit Rührer, Thermometer, 100 ml-Tropftrichter (ohne Druckausgleich), Dreiwege-Schwanzhahn für Inertbegasung und Dimroth-Kühler ausgestattetem 21-Vierhalsrundkolben wurden unter Argon 63,1 g (0,5 mol, getrocknet, mit maximalem Wassergehalt von 0,2%) OXA vorgelegt. Anschliessend wurden 285 ml (222,9 g, 5,43 mol) Acetonitril und 350,2 ml (272,1 g, 1,47 mol) Tri-(n-butyl)amin zugefügt. Die resultierende Suspension wurde 5 Minuten bei 25°C gerührt, und anschliessend wurden innerhalb einer Stunde aus einem Tropftrichter 40 ml (59,3 g, 0,35 mol) SiCl₄ so zugetropft, dass die Innentemperatur im Kolben nicht über 45° C anstieg. Der Ansatz war nach Zugabe des Siliziumtetrachlorides rötlich gefärbt. Man heizte dann das Reaktionsgemisch im Oelbad auf 55° C auf, währenddessen sich der Kolbeninhalt dunkel verfärbte. Die Bildung von OXN wurde mittels GC und Zugabe eines internen Standards (C₁₁-Alkan) direkt aus dem Ansatz bestimmt.

Nach einer Reaktionszeit von 2,5 Stunden stellte sich ein Gleichgewicht von 79% OXN und 15% OXA ein (etwa 6% der erwarteten Gesamtmenge wurden nicht erfasst). Um die Ausbeute an OXN zu erhöhen, wurden zusätzlich 2,2 ml (3,25 g, 19,6 mmol) SiCl₄ unverdünnt innert 10 Minuten zum Ansatz zugetropft, so dass die Gesamtmenge an SiCl₄ dann 62,55 g (0,369 mol, d.h. 0,738 Aeq. SiCl₄ bezogen auf OXA) betrug. Nach einer Gesamtreaktionszeit von 5,5 Stunden wurden etwa 89,4% OXN und 4,4% OXA (etwa 6,2% nicht erfasst) festgestellt. Die Reaktionslösung wurde nach einer Gesamtreaktionszeit von 6,5 Stunden auf 25°C abgekühlt und wie nachfolgend beschrieben aufgearbeitet:

Unter Rühren wurden 4,21 ml (0,234 mol) entionisiertes Wasser zugetropft wobei die Temperatur der Reaktionslösung auf etwa 45°C anstieg. Nach 15 Minuten wurde auf 25°C gekühlt. Nach Entfernung des Dimroth-Kühlers und Austausch des Tropftrichters durch eine Destillationsbrücke wurden alle destillierbaren Substanzen (Lösungsmittel Acetonitril, OXN, Base Tri(n-butyl)-amin und eventuell geringe Mengen an Tri(n-butyl)aminhydrochlorid) flach destilliert.

Die Gesamtmenge Destillat betrug 291,1 g (Soll: 272 g aus 223 g Acetonitril und 49 g OXN).

Zur Aufarbeitung und Isolierung des Tri(n-butyl)aminhydrochlorids wurden zum Destillationsrückstand bei einer Oelbadtemperatur von 90°C innerhalb von 15 Minuten ohne Rühren 237,3 ml 28%ige Natronlauge (2,202 mol NaOH bezogen auf die Menge SiCl₄) zugetropft. Nach etwa 60 Minuten war eine klare Lösung entstanden. Der Kolbeninhalt wurde auf 25°C gekühlt und danach in einen 2 l-Scheidetrichter überführt, wobei mit 60 ml entionisiertem Wasser nachgespült wurde. Im Scheidetrichter erfolgte eine schnelle Phasentrennung. Es wurden 238 g rohes Tri(n-butyl)amin als klares rotbraun gefärbtes Produkt erhalten. Man extrahierte die wässrige Phase dreimal mit je 60 ml tert.Butylmethylether nach. Die Etherphasen wurden vereinigt und unter vermindertem Druck (etwa 400 mbar) bei einer Badtemperatur von 40°C konzentriert. Etwa 1 g rohes Tri(n-butyl)amin wurde erhalten.

Siliziumdioxid wurde durch Einstellung des pH-Wertes auf 6,3 als fester, nahezu farbloser Niederschlag gefällt. Anschliessend wurde dieser Niederschlag durch Filtration abgetrennt, und bis zur Gewichtskontanz getrocknet.

## Patentansprüche

1. Verfahren zur Herstellung eines 5-Cyano-4-C₁₋₆-alkyl-oxazols durch Dehydratisierung eines 5-Carbamoyl-4-C₁₋₆-alkyl-oxazols, **dadurch gekennzeichnet, dass** man die Dehydratisierung mit Siliziumtetrachlorid in Gegenwart eines Amins und in einem organischen Lösungsmittel durchführt.

2. Verfahren nach Anspruch 1, **dadurch gekennzeichnet, dass** der im Ausgangsmaterial und Endprodukt vorhandene C₁₋₆-Alkylrest Methyl oder. Ethyl ist.

3. Verfahren nach Anspruch 1 oder 2, **dadurch gekennzeichnet, dass** das Amin ein aliphatisches Amin oder eine Stickstoff enthaltende heteroaromatische Verbindung ist.

4. Verfahren nach Anspruch 3, **dadurch gekennzeichnet, dass** das aliphatische Amin Triethylamin, Diisopropylethylamin oder Tri(n-butyl)amin ist.

5. Verfahren gemäss Anspruch 3, **dadurch gekennzeichnet, dass** die Stickstoff enthaltende heteroaromatische Verbindung Pyridin oder ein Pyridinderivat ist.

6. Verfahren nach einem der Ansprüche 1 bis 5, **dadurch gekennzeichnet, dass** als organisches Lösungsmittel ein aliphatischer oder cyclischer Ether, ein aliphatisches Nitril, ein aromatischer Kohlenwasserstoff, oder N-Methylpyrrolidon verwendet wird.

7. Verfahren nach Anspruch 6, **dadurch gekennzeichnet, dass** als Lösungsmittel tert. Butylmethylether oder Acetonitril verwendet wird.

8. Verfahren nach einem der Ansprüche 1 bis 7, **dadurch gekennzeichnet, dass** die Dehydratisierung bei Temperaturen von etwa 25°C bis etwa 60°C durchgeführt wird.

9. Verfahren nach einem der Ansprüche 1 bis 8, **dadurch gekennzeichnet, dass** das Molverhältnis Siliziumchlorid:5-Carbamoyl-4-niederalkyl-oxazol von etwa 0,7:1 bis etwa 0,75:1 beträgt.

10. Verfahren nach einem der Ansprüche 1 bis 9, **dadurch gekennzeichnet, dass** das Molverhältnis Amin:5-Carbamoyl-4-niederalkyl-oxazol von etwa 2,7 : 1 bis etwa 2,9 : 1 beträgt.

## Claims

1. A process for the manufacture of a 5-cyano-4-C₁₋₆-alkyl-oxazole by the dehydration of a 5-carbamoyl-4-C₁₋₆-alkyl-oxazole, which process comprises carrying out the dehydration with silicon tetrachloride in the presence of an amine and in an organic solvent.

2. A process according to claim 1, wherein the C₁₋₆-alkyl group present in the starting material and end product is methyl or ethyl,

3. A process according to claim 1 or 2, wherein the amine is an aliphatic amine or a nitrogen-containing heteroaromatic compound.

4. A process according to claim 3, wherein the aliphatic amine is triethylamine, diisopropylethylamine or tri(n-butyl)amine.

5. A process according to claim 3, wherein the nitrogen containing heteroaromatic compound is pyridine or a pyridine derivative.

6. A process according to any one of claims 1 to 5, wherein an aliphatic or cyclic ether, an aliphatic nitrile, an aromatic hydrocarbon or N-methylpyrrolidone is used as the organic solvent.

7. A process according to claim 6, wherein tert. butyl methyl ether or acetonitrile is used as the solvent.

8. A process according to any one of claims 1 to 7, wherein the dehydration is carried out at temperatures from about 25°C to about 60°C.

9. A process according to any one of claims 1 to 8, wherein the silicon chloride:5-carbamoyl-4-lower alkyl oxazole molar ratio is from about 0.7:1 to about 0.75:1.

10. A process according to any one of claims 1 to 9, wherein the amine:5-carbamoyl-4-lower alkyl-oxazole molar ratio is from about 2.7:1 to about 2.9:1.

## Revendications

1. Procédé pour la préparation d'un 5-cyano-4-alkyl(C₁-C₆)-oxazole par déshydratation d'un 5-carbamoyl-4-alkyl(C₁-C₆)-oxazole, **caractérisé en ce qu'**on effectue la déshydratation avec du tétrachlorure de silicium en présence d'une amine et dans un solvant organique.

2. Procédé selon la revendication 1, **caractérisé en ce que** le radical alkyle en C₁-C₆, présent dans le produit de départ et dans le produit final est le groupe méthyle ou éthyle.

3. Procédé selon la revendication 1 ou 2, **caractérisé en ce que** l'amine est une amine aliphatique ou un composé hétéroaromatique contenant de l'azote.

4. Procédé selon la revendication 3, **caractérisé en ce que** l'amine aliphatique est la triéthylamine, la diisopropyléthylamine ou la tri(n-butyl)amine.

5. Procédé selon la revendication 3, **caractérisé en ce que** le composé hétéroaromatique contenant de l'azote est la pyridine ou un dérivé de pyridine.

6. Procédé selon l'une quelconque des revendications 1 à 5, **caractérisé en ce qu'**on utilise comme solvant organique un éther aliphatique ou cyclique, un nitrile aliphatique, un hydrocarbure aromatique ou la N-méthylpyrrolidone.

7. Procédé selon la revendication 6, **caractérisé en ce qu'**on utilise comme solvant l'éther tert-butylméthylique ou l'acétonitrile.

8. Procédé selon l'une quelconque des revendications 1 à 7, **caractérisé en ce qu'**on effectue la déshydratation à des températures d'environ 25°C à environ 60°C.

9. Procédé selon l'une quelconque des revendications 1 à 8, **caractérisé en ce que** le rapport molaire chlorure de silicium:5-carbamoyl-4-alkyl(inférieur)-oxazole va d'environ 0,7:1 à environ 0,75:1.

10. Procédé selon l'une quelconque des revendications 1 à 9, **caractérisé en ce que** le rapport molaire amine:5-carbamoyl-4-alkyl(inférieur)-oxazole va d'environ 2,7:1 à environ 2,9:1.
